# EUROPEAN PATENT SPECIFICATION

(11) **EP 1 161 270 B1**
(45) Date of publication and mention of the grant of the patent: **10.01.2007**
(21) Application number: 00916414.6
(22) Date of filing: 16.03.2000
(51) Int. Cl.: A61M 1/36

(54) **SYSTEM FOR PROCESSING BLOOD**
BLUTBEHANDLUNGSSYSTEM
SYSTEME DE TRAITEMENT DU SANG

(30) Priority: 17.03.1999 US 271627
(43) Date of publication of application: 12.12.2001
(73) Proprietor: HAEMONETICS CORPORATION, Braintree Massachusetts 02184 (US)
(72) Inventor: HEADLEY, Thomas, D., Wellesley, MA 02181 (US)
(74) Representative: Greene, Simon Kenneth
(86) International application number: PCT/US2000/006945
(87) International publication number: WO 2000/054824

(56) References cited:
- EP-A- 0 349 188
- EP-A- 0 852 151
- US-A- 5 733 253

## Description

### Technical Field

This invention generally relates to systems for processing blood and other biological fluids.

By way of background, reference may be made to US-A-5,849,203, which relates to methods of accumulating separated blood components in a rotating chamber for collection.

A system for processing blood as defined in the preamble of claim 1 is disclosed in US-A- 5 733 253.

### Summary of the Invention

The present invention is directed to systems for collecting a separated blood component that is free of white blood cells (WBCs), and in particular, systems and methods that are simple to use and that reduce the need for human intervention. A disposable set is provided with (i) a port (e.g., a cannula inserted into a vein of the donor) for permitting whole blood to flow from a donor into the disposable set, (ii) a WBC filter, (iii) a separation container wherein the whole blood is separated into components (for example, a centrifuge rotor or other separation means), (iv) a blood-component container for storing a separated blood component, and (v) tubing connecting the port, the filter, the separation container and the blood-component container. The port acts as an inlet allowing whole blood to flow into the disposable set. The WBC filter is located between the port (i.e., the inlet) and the separation container and is capable of filtering while blood cells from the whole blood. The inlet port is connected to the donor, and whole blood is drawn from the donor through the port. The whole blood is directed from the port through the WBC filter to the separation container, so that white blood cells are filtered from the whole blood before entering the separation container. After the whole blood is separated into a first component and a second component, one of the first and second components is directed from the separation container to the blood-component container.

A preferred embodiment of the present invention is directed to systems for collecting red blood cells from a donor while returning plasma to the donor. Whole blood is removed from a donor, white blood cells (WBCs) are filtered from the whole blood, and the filtered blood is directed to a centrifuge rotor, where the blood is separated into red blood cells (RBCs) and plasma. The plasma may be returned to the donor during the same procedure in which whole blood is drawn from the donor. At one or more times during the collection process, when all the rotor has been emptied of plasma, the red blood cells are urged from the rotor and collected in one or more RBC containers.

In a preferred embodiment, the system for collecting red blood cells includes a disposable set as set forth above, and having a centrifuge rotor for the separation container and an RBC container for the blood-component container. In preferred embodiments, the port also acts as an outlet, so as to permit the return of a separated blood component to the donor; alternatively, a separate outlet port may be provided. The WBC filter is located between the port and the centrifuge rotor. The RBC container is connected to a branch of the tubing leading from the rotor.

Preferably, the centrifuge rotor has a variable total volume. The rotor preferably includes a fixed portion, a rotatable portion and a rotary seal providing a seal between the fixed and rotatable portions, wherein the tubing is connected to the rotor's fixed portion. In order to create a variable total volume, the centrifuge rotor may include in its rotatable portion an elastic diaphragm, which stretches to increase the total volume of the rotor. In such an embodiment, an internal wall is preferably included, for separating the diaphragm from the rotor's fixed portion.

The system also includes a control unit having a spinner in which the rotor may be held. The control unit may include means for varying the volume of the centrifuge rotor by changing the pressure of a gas adjacent the elastic diaphragm. Preferably, a vacuum may be applied to the elastic diaphragm by the control unit, so as to draw blood into the rotor.

In a preferred system, the rotor is placed in the spinner, and the port is connected to the donor. After the rotor is placed in the spinner, whole blood may be drawn from the donor through the port. The whole blood is directed from the port through the WBC filter to the rotor, so that white blood cells are filtered from the whole blood. While the donor is still connected to the port, the spinner rotates the rotor so as to separate the whole blood into plasma and red blood cells. The plasma is urged out of the rotor first, while the rotor is still spinning, to be directed back to the donor. The second component is directed from the rotor to the RBC container.

In one embodiment, an inlet port may be used for drawing whole blood, and a separate outlet port may be used for returning the plasma component. In a preferred embodiment, a port is used to draw whole blood and to return the plasma component; in this embodiment, a temporary storage container is used to hold the plasma while whole blood is being collected, and the plasma is returned when the red blood cells are being processed by and urged from the rotor.

### Brief Description of the Drawings

FIG. 1 shows a system according to the present invention.
FIG. 2 shows a sectional view of a rotor that may be used in the system of the present invention.
FIG. 3 shows a disposable set that may be used in the system shown in FIG. 1.
FIG. 4 shows an alternative disposable set.
FIG. 5 shows the arrangement of steps in a cycle of a process for collecting red blood cells using the disposable set of FIG. 4.

### Description of Specific Embodiments

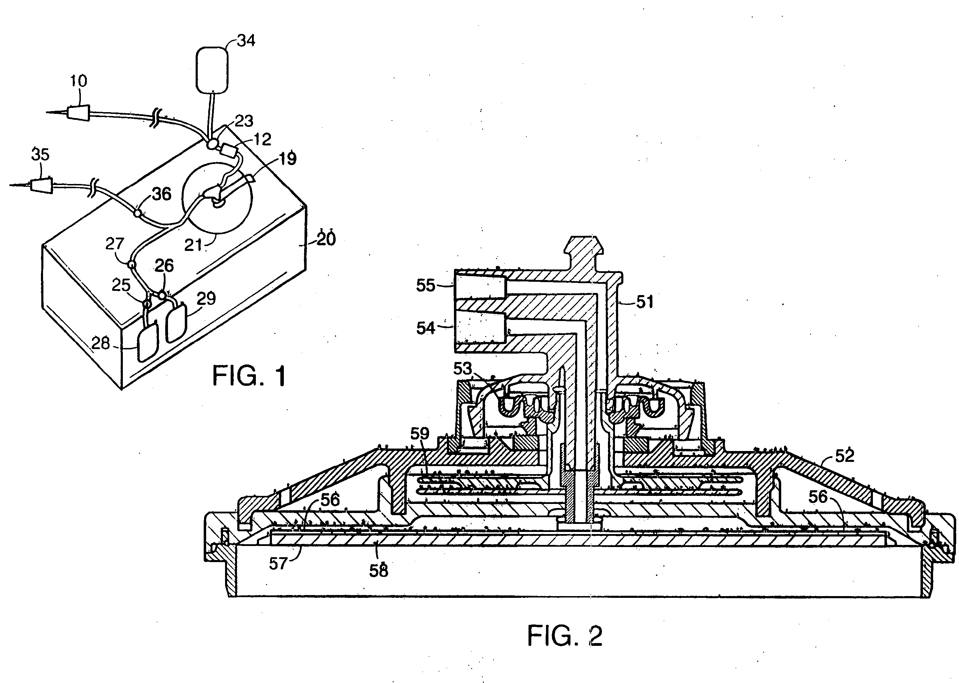

FIG. 1 shows an embodiment of a system according to the present invention. The system uses many of the components and features described in U.S. Patent Nos. 5,733,253 issued March 31, 1998 to Headley and Powers, which relates to a fluid separation system, and 5,651,766 issued July 29, 1997 to Kingsley, Headley and Halpern, which relates to a blood collection and separation system.

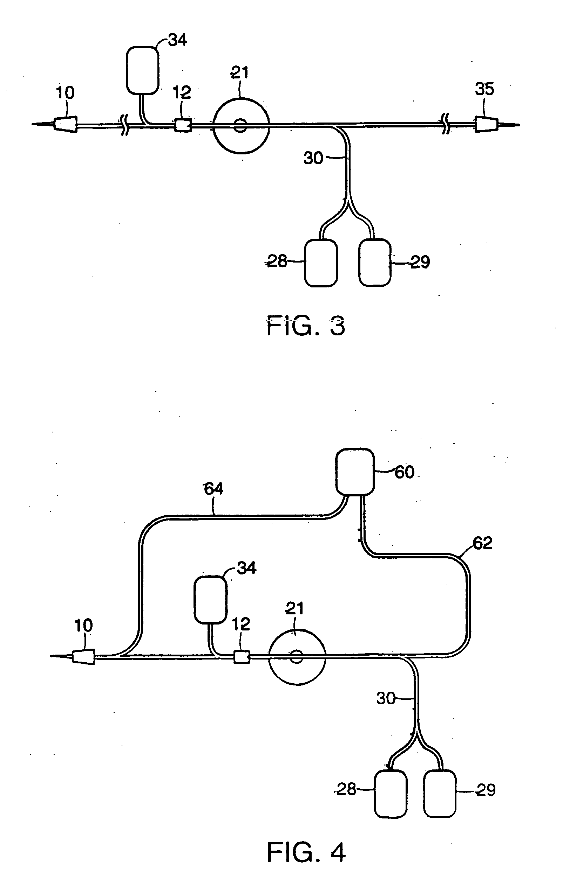

The system includes a disposable set (such as the one shown in FIG. 3) and a control unit that controls the disposable set. The disposable set includes an inlet port **10,** e.g., a cannula, which may act as a connector for attachment to a shunt or other arrangement for permitting whole blood to enter the disposable set from the donor. Tubing-connects the various components of the disposable set. The whole blood passes through some of the tubing and flows into the centrifuge rotor **21** mounted in a control unit **20.** A WBC filter **12** is located in the tubing between the inlet port **10** and the rotor **21.** This filter **12** filters white blood cells from the whole blood. Filtering the whole blood before separation is easier than filtering the red blood cells after separation, because of the lower density of the whole blood. Filtering the whole blood before separation results in the white blood cells being removed from the plasma as well as the red blood cells.

The filtered blood is spun in the rotor at a sufficiently high speed so as to cause the blood to separate into plasma and red blood cells. The plasma is returned to the donor through an outlet port **35** (e.g., a cannula), while blood is being drawn from the donor. The red blood cells are collected in one or more RBC containers **28, 29**.

An anticoagulant bag **34** is preferably connected to the tubing between the inlet port **10** and the filter **12,** so as to provide anticoagulant to the whole blood being drawn. A metering valve **23** on and controlled by the control unit may be used to meter the anticoagulant from the anticoagulant-container **34** into the whole blood being drawn from the inlet port **10.** In lieu of or in addition to valve **23,** a peristaltic pump or other flow-inducing arrangement may be used to add the anticoagulant to the whole blood and/or help draw blood from the donor. In a preferred embodiment, the tubing may be modified so that the anticoagulant is added to the whole blood coming from the donor at a point in the tubing much closer to the inlet port **10**. (In addition to--or in lieu of--the anticoagulant, a replacement fluid, such as saline, may be added to the whole blood being drawn. After the whole blood has been centrifuged, plasma is returned to the donor along with the replacement fluid. The replacement fluid provides the donor with a volume of fluid to replace the volume of red blood cells that are collected. Adding replacement fluid to the whole blood has an anticoagulating effect. Anticoagulant may be added to the replacement fluid to provide more anticoagulation.)

The rotor **21** preferably has a variable total volume and two ports to permit the introduction of blood into the rotor at the same time a blood component is being removed. In a preferred embodiment, the rotor is of the type shown in Figures 10-15 or Figures 23-27 in above-referenced U.S. Patent No. 5,733,253.

FIG. 2 is a sectional view of a rotor which may be used in the system shown in FIG.1, and which is a variation on the rotor shown in Figures 10-15 of U.S. Patent No. 5,733,253. The rotor includes a fixed portion **51,** which does not rotate and which (as shown in FIG. 1) is held in place by a brace **19** on the control unit 20; a rotatable portion **52,** which is held and spun by a chuck in the control unit; and a rotary seal 53, which maintains a seal between the fixed portion and the rotatable portion. The rotary seal preferably works in the same manner as the rotary seal shown in Figures 38 and 39 of U.S. Patent No. 5,733,253: The sealing force applied by the rotary seal **53** is not substantially affected by changes in air pressure within the rotor. The rotary seal is mounted on a base, which may be part of the rotor's fixed portion **51.** The rotary seal **53** includes first and second rigid seal members, which surround the axis of rotation, and which spin in relation to each other. As set forth in Patent No. 5,733,253, the first rigid seal member and the base define an annular passage between them, and the first rigid seal member has a step portion which extends radially across the annular passage. A spring member surrounds the rotary seal's axis of rotation and is connected to the base and to the first rigid seal member, so that the spring member applies a force pressing the first rigid seal member against the second rigid seal member. A flexible seal member surrounds the axis of rotation and prevents fluid flow between the first rigid seal member and the base. The flexible seal member extends across the annular passage such that pressure from the annular passage exerts forces on the flexible seal member and the step portion which cancel each other, so that the force with which the spring member presses the first rigid seal member against the second rigid seal member is not substantially affected by pressure within the annular passage.

The rotor's fixed portion includes a rotor inlet **54** and a rotor outlet **55**, which are connected by tubing with the rest of the disposable set. The tubing of the disposable set has a portion that provides whole blood to the inlet **54** of the rotor **21** and another portion that provides blood components from the rotor's outlet **55** to outlet port **35** and to the RBC-collection containers **28, 29.** The rotor inlet **54** leads to a fluid passage down the fixed portion **51** to a pair of channels **56,** which are in the rotor's rotatable portion **52.** The channels **56** permit the blood that has come from the donor and passed through the WBC filter to flow to the outer perimeter of the rotor's interior volume. Because the rotor's interior volume is defined in part by a flexible, preferably elastic, diaphragm, the rotor has a variable total volume.

Preferably, only two such inflow channels 56 providing fluid communication from the rotor inlet **53** are used, in order to minimize the amount of blood trapped in the rotor when the process is completed, and these two channels are preferably disposed 180° from each other, in order to maintain balance in the rotor. In alternative embodiments, one or more than two inflow channels **56** may be used. The rotor includes an interior wall **58,** which together with the flexible diaphragm **57** fully define the rotor's processing chamber. The interior wall **58** includes grooves on its bottom surface (not shown), which permit flow of blood components out of the rotor's processing chamber. In a preferred embodiment, the interior wall **58** includes two of these outflow grooves arranged 180° to each other and at 90° to the inflow channels **56.** The outflow grooves lead to holes which pass up through the interior wall **58** to the region adjacent a collector **59.** These holes (not shown) provide fluid communication between the outflow grooves and the collector **59,** which is part of the rotor's fixed portion **51.** The collector **59** collects the blood components flowing out of the processing chamber and directs the blood components up through a vertical passage to the rotor outlet **55.**

Blood may be drawn into the rotor **21** by creating a vacuum in the chuck that holds the rotor **21.** Alternatively, a peristaltic pump or other flow-inducing arrangement may be used, in lieu of or in addition to valve **23,** to draw the whole blood from the port **10** into the rotor **21.**

When a sufficient amount of whole blood has entered the rotor **21,** the rotor is spun sufficiently fast so as to separate the blood into plasma and red blood cell components. After the blood has separated into plasma and red blood cells, valve **36** is opened, and plasma (and replacement fluid) is urged from the rotor **21** and is directed back to the donor through the outlet port **35.** Plasma may be removed from the rotor as filtered blood is continuing to be introduced into the rotor.

Because the plasma component of the blood is being returned to the donor, two units of red blood cells may ordinarily be collected from the donor. In accordance with industry practice, the two units of red blood cells may be stored in two separate containers **28** and **29.** In an alternative embodiment, a single container may be used, instead of two containers.

When it is determined that enough red blood cells have been collected, or when the rotor is substantially filled with red blood cells, whatever plasma remains in the rotor is forced from the rotor **21.** When substantially all of the plasma has been removed from the rotor, valve **36** is closed and valves **27** and **25** are opened; the red blood cells are urged from the rotor and directed through valves **27** and **25** into the **RBC-storage** container **28**. Since the white blood-cells have already been filtered from the whole blood by the WBC filter **12,** it is unnecessary in the present system to include a WBC filter between the rotor **21** and the RBC-storage containers **28** and **29.**

The RBC-storage containers **28** and **29** are preferably bags and preferably contain RBC preservative (or storage solution). The RBC-storage bags **28** and **29** preferably hold a unit of red blood cells each. When a unit of red blood cells have been delivered to RBC-storage container **28,** valve **25** is closed and valve **26** opened, thereby permitting the red blood cells to flow into RBC-storage bag **29.** Valves **25, 26, 27** and **36,** along with valve **23,** are controlled by the control unit 20. (This system may be modified so that platelets are separately collected in the manner set forth in concurrently filed application serial no. 09/271,061 for a "System and Method for Collecting Platelets and Other Blood Components".

Depending on the maximum volume of the centrifuge rotor **21** used in relation to the total volume of the two RBC-storage containers **28, 29** used, it may be necessary to go through several cycles of filling the rotor, separating the blood into RBC and plasma components and urging plasma from the rotor until the rotor becomes filled with red blood cells, and at that point, urging the red blood cells from the rotor into one or more of the RBC-storage containers, in order to obtain desired amount (preferably two units) of red blood cells. Once the storage bags **28, 29** are filled with red blood cells, the storage bags are removed from the rest of the disposable set by cutting and heat sealing the tubes leading to the storage bags.

The process described hereinabove is highly automated compared to the prior-art methods of processing blood. A blood donation technician installs the disposable set into the control unit **20** and inserts the inlet and outlet ports **10, 35** into the donor's arm. The technician, of course, also removes the cannulas from the donor's arms, and cuts and heat seals the tubing leading to the storage bags. The remaining steps of the process may be performed by the control unit: controlling the valves (and any pumps) to direct the flow of blood or blood components; determining when the rotor is sufficiently full, spinning the rotor; urging blood components from the rotor; and determining when the rotor has been emptied of a blood component.

As noted above, in a preferred embodiment of the control unit, each of valves **23, 25, 26, 27** and **36** are controlled by the control unit **20,** as is the speed that the rotor **21** is spun. Several of the valves **23, 25, 26, 27** and **36** may be combined into a single valve mechanism. The preferred embodiment of the rotor **21** has a stretchable elastic diaphragm **57** that defines the interior volume of the rotor **21.** As discussed in the above-referenced U.S. Patent Nos. 5,733,253 and 5,651,766, the elastic diaphragm (item **57** of FIG. 2) permits the total volume of the rotor **21** to be varied by varying the air pressure applied to the elastic diaphragm; this air pressure is also preferably controlled by the control unit so as to vary and control the total volume of the rotor **21.** This air pressure may also be used to force fluid from the rotor **21** by increasing the air pressure sufficiently or to draw fluid into the rotor **21** by decreasing air pressure sufficiently.

The disposable set shown in FIGS. 1 and 3, as well as the control unit **20** shown in FIG. 1, may in a preferred embodiment be modified to use a single cannula or port to draw whole blood from the donor and return plasma to the donor. Such a disposable set is shown in FIG. 4. A temporary storage container **60** holds the separated plasma component for return to the donor. The separated plasma component is urged by the control unit from the rotor **21** through a portion **62** of the tubing to the temporary storage container **60.** The control unit preferably urges the plasma from the rotor by increasing the gas pressure against the rotor's elastic membrane. Once plasma begins flowing from the rotor, the continued introduction of whole blood to the rotor tends to continue forcing separated plasma out of the rotor. After a desired amount of whole blood has been collected from the donor through port 10 (preferably when the rotor is almost full of red blood cells), the collection of whole blood is suspended, and plasma may be urged from the temporary storage container **60** through another portion **64** of the tubing to the port **10** and the donor. The control unit may be provided with means, such as a peristaltic pump working on tubing portion **64,** for effecting and controlling the flow of plasma from the temporary storage container **60** to the port **10.** The rest of the disposable set may be same as the disposable set shown in FIG. 3, including a WBC filter **17** in another portion of the tubing leading from the port **10** to the rotor **21,** so as to permit the white blood cells to be filtered from the whole blood before it is separated; an anticoagulant container **34** may be attached to this same portion of the tubing. In a preferred embodiment, the anticoagulant container may be connected to a point in the tubing closer to the port **10,** so as to introduce the anticoagulant to the whole blood as soon as possible after it is drawn from the donor.

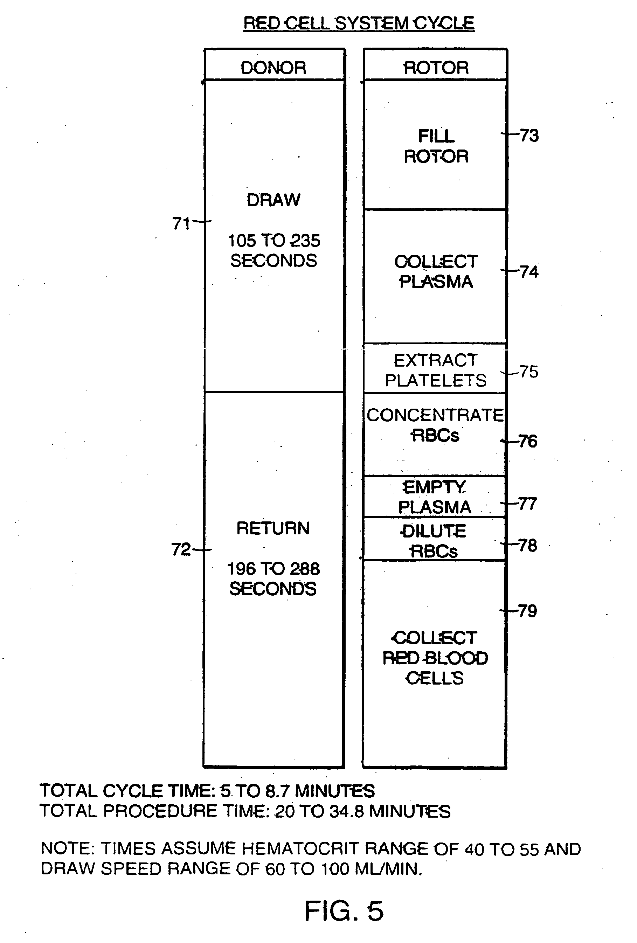

The process of collecting red blood cells using the disposable set shown in FIG. 4 may involve several cycles of collecting whole blood from the donor and returning the separated plasma component to the donor. FIG. 5 shows an outline of steps in one such cycle. Each cycle is divided up into two periods: a first period **71** in which whole blood is drawn from the donor, i.e., a draw period (which in one embodiment lasts between 105 and 235 seconds), and a second period **72** in which plasma is returned to the donor, i.e., a return period (which lasts between 196 and 288 seconds). During the draw period **71,** the rotor--item **21** in FIG. 4--is filled with whole blood from the donor (step **73**), and the rotor is spun to separate the blood into plasma and RBC components. Plasma is urged from the rotor to the temporary container **60** (step **74**--of course, some of these steps may overlap). In an optional step (step **75**), the platelets may be separately collected in the manner set forth in concurrently filed application serial no. 09/271,601, for a "System and Method for Collecting Platelets and Other Blood Components".

During the return period **72,** the red blood cells are concentrated, by increasing the rotational speed of the centrifuging rotor (step **76**), and the remaining plasma is removed from the rotor **21** to the temporary container **60** (step **77**). An RBC-storage solution may be added to the red blood cells, diluting the red blood cells (step **78**). In the final step of the cycle (step **79**), the red blood cells are directed through the portion **30** of the tubing to one of the two storage containers **28** or **29.**

When the red blood cells have been moved from the rotor to the storage containers, and when the plasma in the temporary container has been returned to the donor, the cycle may start again with the draw period **71** and the rotor filling step **73.** In one embodiment, the total cycle time lasts from 5 to 8.7 minutes (assuming a hematocrit range of 40 to 55 and a draw speed range of 60 to 100 ml/min). Four cycles may be executed, for a total procedure time of 20 to 34.8 minutes.

The disposable sets shown in FIGS. 3 and 4--the storage bags **28, 29,** filter **12,** centrifuge rotor **21** and the tubing--may be configured in several ways. The tubing may consist solely of tubes which may be squeezed by the control unit to direct flow or to pump in a peristaltic manner. Alternatively, the tubing may contain special valving or pumping components (such as a pumping/valving cassette) which may be acted on by the control unit. The phrase "flow-control arrangement" refers herein to any structure or system for controlling or causing flow of fluid between the various components of the systems of the present invention.

The control unit shown in FIG. 1 and the disposable set shown in FIGS. 1 and 3 may also be modified, by substituting a plasma collection container for the outlet port **35,** to permit the collection of plasma that is free of white blood cells, as well as the collection of red blood cells that are free of white blood cells.

## Claims

1. A disposable set for use in a control unit (20) for processing blood, the disposable set comprising: a port (10); a filter (12) located between the port and a separation container (21); a blood-component container (28, 29); and tubing (30) connecting the port, the filter, the separation container and the blood-component container; **characterised in that** the filter (12) is arranged to filter white blood cells from whole blood before separation.

2. A disposable set according to claim 1 wherein the separation container is a variable-volume centrifuge rotor (21) having a fixed portion (51), a rotatable portion (52) and a rotary seal (53) providing a seal between the fixed and the rotatable portions.

3. A disposable set according to claim 1 wherein it further comprises: a temporary storage container (60); and tubing connecting the port, the filter, the rotor, the container and the temporary storage container; the separation container comprising a centrifuge rotor, the blood-component container comprising a RBC container (28, 29) and the filter being located between the port and the centrifuge rotor, the centrifuge rotor having a fixed portion, a rotatable portion and a rotary seal providing a seal between the fixed and the rotatable portions, and having a variable total volume.

4. A disposable set according to claim 3 wherein the RBC container is connected to a branch of the tubing between the rotor and the temporary storage container.

5. A disposable set according to claim 3 wherein the centrifuge rotor is provided with a flexible diaphragm (57) which defines the volume of the rotor.

6. A disposable set according to claim 1 wherein: the port permits the introduction of blood previously-collected from a donor into the disposable set; whole blood is separated into components in the separation container; the filter is located between the port and the separation container, and is capable of filtering white blood cells, and the blood-component container is for storing a separated blood component, the blood-component container being in fluid communication with the separation container; and, in combination with the disposable set, a control unit (20) having a flow-control arrangement (23, 25, 26, 27, 36) which urges a blood component from the separation container to the blood-component container after the blood has been separated.

7. A disposable set according to claim 6 wherein the separation container is a variable volume centrifuge rotor, and the control unit further comprises means for spinning the rotor and for varying the volume of the rotor (97).

8. A disposable set according to claim 7 wherein the centrifuge rotor comprises an elastic diaphragm (97) which defines the volume of the rotor, and the control unit comprises means for varying gas pressure adjacent the elastic diaphragm.

9. A disposable set according to claim 8 wherein the means for varying gas pressure comprises means for applying a vacuum to the elastic diaphragm, so as to draw blood into the rotor.

10. A disposable set according to claim 6 wherein the separation container comprises a centrifuge rotor having a variable total volume; the filter is located in a fluid path between the port and the rotor; the blood-component container comprises an RBC container in fluid communication with the centrifuge rotor; and the control unit having a spinner that holds the rotor, the spinner being able to spin the rotor so as to separate blood into blood components; and the flow-control arrangement urges a blood component from the rotor while the rotor is being spun.

11. A disposable set according to claim 10 wherein the control unit further comprises means for varying the volume of the rotor.

12. A disposable set according to claim 11 wherein the centrifuge rotor comprises an elastic diaphragm which defines the volume of the rotor, and the control unit comprises means for varying gas pressure adjacent the elastic diaphragm.

13. A disposable set according to claim 12 wherein the means for varying gas pressure comprises means for applying a vacuum to the elastic diaphragm, so as to draw blood into the rotor.

## Patentansprüche

1. Einwegsatz zur Verwendung in einem Kontrollgerät (20) zum Verarbeiten von Blut, wobei der Einwegsatz Folgendes aufweist: einen Stutzen (10); einen Filter (12), der zwischen dem Stutzen und einem Separationsgehäuse (21) angeordnet ist; einen Blutkomponentenbehälter (28, 29); und eine Leitungsanlage (30), welche den Stutzen, den Filter, das Separationsgehäuse und den Blutkomponentenbehälter verbindet; **dadurch gekennzeichnet, dass** der Filter (12) dafür eingerichtet ist, vor der Separation weiße Blutzellen aus dem Vollblut zu filtern.

2. Einwegsatz nach Anspruch 1, wobei das Separationsgehäuse ein Zentrifugenrotor (21) mit veränderbarem Volumen ist, der einen festen Abschnitt (51), einen drehbaren Abschnitt (52) und eine Gleitringdichtung (53) aufweist, die eine Abdichtung zwischen den festen und drehbaren Abschnitten bildet.

3. Einwegsatz nach Anspruch 1, der ferner folgendes aufweist: einen Zwischenlagerbehälter (60); und eine Leitungsanlage, welche den Stutzen, den Filter, den Rotor, den Behälter und den Zwischenlagerbehälter verbindet; wobei das Separationsgehäuse einen Zentrifugenrotor aufweist, der Blutkomponentenbehälter einen Behälter (28, 29) für rote Blutzellen aufweist und der Filter zwischen dem Stutzen und dem Zentrifugenrotor angeordnet ist, wobei der Zentrifugenrotor einen festen Abschnitt, einen drehbaren Abschnitt und eine Gleitringdichtung aufweist, die eine Abdichtung zwischen den festen und drehbaren Abschnitten bildet, und ein veränderbares Gesamtvolumen aufweist.

4. Einwegsatz nach Anspruch 3, wobei der Behälter für rote Blutzellen an einen Zweig der Leitungsanlage zwischen dem Rotor und dem Zwischenlagerbehälter angeschlossen ist.

5. Einwegsatz nach Anspruch 3, wobei der Zentrifugenrotor mit einer flexiblen Membrane (57) ausgestattet ist, die das Volumen des Rotors bestimmt.

6. Einwegsatz nach Anspruch 1, wobei der Stutzen das Einleiten von zuvor gesammeltem Blut von einem Spender in den Einwegsatz ermöglicht; Vollblut im Separationsgehäuse in Komponenten getrennt wird; der Filter zwischen dem Stutzen und dem Separationsgehäuse angeordnet ist und zum Filtern weißer Blutzellen befähigt ist, und der Blutkomponentenbehälter zum Lagern einer abgeschiedenen Blutkomponente dient, wobei der Blutkomponentenbehälter mit dem Separationsgehäuse in Fluidverbindung steht; und wobei ein Kontrollgerät (20) in Verbindung mit dem Einwegsatz eine Strömungskontrolleinrichtung (23, 25, 26, 27, 36) aufweist, die nach dem Trennen des Bluts eine Blutkomponente vom Separationsgehäuse zum Blutkomponentenbehälter treibt.

7. Einwegsatz nach Anspruch 6, wobei das Separationsgehäuse ein Zentrifugenrotor mit veränderbarem Volumen ist und das Kontrollgerät ferner Mittel zum schnellen Drehen des Rotors und zum Ändern des Volumens des Rotors (57) aufweist.

8. Einwegsatz nach Anspruch 7, wobei der Zentrifugenrotor eine elastische Membrane (57) aufweist, die das Volumen des Rotors bestimmt, und das Kontrollgerät Mittel zum Ändern des an die elastische Membrane angrenzenden Gasdrucks aufweist.

9. Einwegsatz nach Anspruch 8, wobei das Mittel zum Ändern des Gasdrucks Mittel zum Aufbringen eines Unterdrucks auf die elastische Membrane aufweist, um Blut in den Rotor zu saugen.

10. Einwegsatz nach Anspruch 6, wobei das Separationsgehäuse einen Zentrifugenrotor mit veränderbarem Gesamtvolumen aufweist; das Filter in einem Strömungsweg zwischen dem Stutzen und dem Rotor angeordnet ist; der Blutkomponentenbehälter einen Behälter für rote Blutzellen aufweist, der mit dem Zentrifugenrotor in Fluidverbindung steht; und das Kontrollgerät ein Schleuderrad aufweist, das den Rotor aufnimmt, wobei das Schleuderrad den Rotor so schnell drehen kann, dass Blut in Blutkomponenten zerlegt wird; und die Strömungskontrolleinrichtung eine Blutkomponente aus dem Rotor treibt, während der Rotor schnell gedreht wird.

11. Einwegsatz nach Anspruch 10, wobei das Kontrollgerät ferner Mittel zum Ändern des Volumens des Rotors aufweist.

12. Einwegsatz nach Anspruch 11, wobei der Zentrifugenrotor eine elastische Membrane aufweist, die das Volumen des Rotors bestimmt, und das Kontrollgerät Mittel zum Ändern des an die elastische Membrane angrenzenden Gasdrucks aufweist.

13. Einwegsatz nach Anspruch 12, wobei das Mittel zum Ändern des Gasdrucks Mittel zum Aufbringen eines Unterdrucks auf die elastische Membrane aufweist, um Blut in den Rotor zu saugen.

## Revendications

1. Ensemble jetable à utiliser dans une unité de commande (20) de traitement du sang, l'ensemble jetable comprenant : un orifice (10) ; un filtre (12) situé entre l'orifice et un récipient de séparation (21) ; un récipient de composant sanguin (28, 29) ; et des tubes (30) reliant l'orifice, le filtre, le récipient de séparation et le récipient de composant sanguin ; **caractérisé en ce que** le filtre (12) est conçu pour filtrer les leucocytes du sang entier avant la séparation.

2. Ensemble jetable selon la revendication 1, dans lequel le récipient de séparation est un rotor centrifuge (21) à volume variable ayant une partie fixe (51), une partie pivotante (52) et un joint rotatif (53) fournissant un joint étanche entre les parties fixe et pivotante.

3. Ensemble jetable selon la revendication 1, comprenant en outre: un récipient de stockage temporaire (60) ; et des tubes reliant l'orifice, le filtre, le rotor, le récipient et le récipient de stockage temporaire; le récipient de séparation comprenant un rotor centrifuge, le récipient de composant sanguin comprenant un récipient de globules rouges (28, 29) et le filtre étant situé entre l'orifice et le rotor centrifuge, le rotor centrifuge ayant une partie fixe, une partie pivotante et un joint rotatif fournissant un joint étanche entre les parties fixe et pivotante et ayant un volume total variable.

4. Ensemble jetable selon la revendication 3, dans lequel le récipient de globules rouges est relié à une branche de tube entre le rotor et le récipient de stockage temporaire.

5. Ensemble jetable selon la revendication 3, dans lequel le rotor centrifuge est pourvu d'une membrane flexible (57) qui définit le volume du rotor.

6. Ensemble jetable selon la revendication 1, dans lequel l'orifice permet l'introduction de sang précédemment recueilli d'un donneur dans l'ensemble jetable ; le sang entier est séparé en composants dans le récipient de séparation ; le filtre est situé entre l'orifice et le récipient de séparation et est en mesure de filtrer les leucocytes et le récipient de composant sanguin est conçu pour stocker un composant sanguin séparé, le récipient de composant sanguin étant en communication fluidique avec le récipient de séparation ; et, en combinaison avec l'ensemble jetable, une unité de commande (20) ayant un agencement de commande du flux (23, 25, 26, 27, 36) qui évacue un composant sanguin du récipient de séparation vers le récipient de composant sanguin une fois le sang séparé.

7. Ensemble jetable selon la revendication 6, dans lequel le récipient de séparation est un rotor centrifuge à volume variable et l'unité de commande comprend en outre des moyens pour faire tourner le rotor et pour faire varier le volume du rotor (97).

8. Ensemble jetable selon la revendication 7, dans lequel le rotor centrifuge comprend un diaphragme élastique (97) qui définit le volume du rotor et l'unité de commande comprend des moyens de variation de la pression du gaz adjacents à la membrane élastique.

9. Ensemble jetable selon la revendication 8, dans lequel les moyens de variation de la pression du gaz comprennent des moyens pour exercer un vide sur la membrane élastique, de façon à tirer du sang dans le rotor.

10. Ensemble jetable selon la revendication 6, dans lequel le récipient de séparation comprend un rotor centrifuge ayant un volume total variable ; le filtre est situé dans un passage de fluide entre l'orifice et le rotor ; le récipient de composant sanguin comprend un récipient de globules rouges en communication fluidique avec le rotor centrifuge ; et l'unité de commande ayant un séparateur centrifuge supportant le rotor, le séparateur centrifuge pouvant faire tourner le rotor de façon à séparer le sang en composants sanguins ; et l'agencement de commande du flux évacuant un composant sanguin hors du rotor lors de la rotation du rotor.

11. Ensemble jetable selon la revendication 10, dans lequel l'unité de commande comprend en outre des moyens de variation du volume du rotor.

12. Ensemble jetable selon la revendication 11, dans lequel le rotor centrifuge comprend une membrane élastique qui définit le volume du rotor et l'unité de commande comprend des moyens de variation de pression du gaz adjacents au diaphragme élastique.

13. Ensemble jetable selon la revendication 12, dans lequel les moyens de variation de pression du gaz comprennent des moyens pour appliquer un vide sur la membrane élastique, de façon à tirer du sang dans le rotor.
